# EUROPÄISCHE PATENTSCHRIFT

(11) **EP 2 552 405 B1**
(45) Veröffentlichungstag und Bekanntmachung des Hinweises auf die Patenterteilung: **23.08.2017**
(21) Anmeldenummer: 11714246.3
(22) Anmeldetag: 30.03.2011
(51) Int. Cl.: A61J 3/00, A61L 31/16, B05D 3/04, A61L 27/34, A61L 27/54, A61K 9/00, A61K 9/51, A61L 17/00

(54) **VERFAHREN ZUR HERSTELLUNG EINES DRUG-DELIVERY-SYSTEMS AUF DER BASIS VON POLYELEKTROLYTKOMPLEXEN**
METHOD FOR PRODUCING A DRUG DELIVERY SYSTEM ON THE BASIS OF POLYELECTROLYTE COMPLEXES
PROCÉDÉ DE PRÉPARATION D'UN SYSTÈME D'ADMINISTRATION DE MÉDICAMENT À BASE DE COMPLEXES DE POLYÉLECTROLYTES

(30) Priorität: 01.04.2010 DE 102010003615
(43) Veröffentlichungstag der Anmeldung: 06.02.2013
(73) Patentinhaber: Leibniz-Institut für Polymerforschung Dresden e.V., 01069 Dresden (DE)
(72) Erfinder: MÜLLER, Martin, 01328 Dresden (DE)
(74) Vertreter: Rauschenbach, Marion
(86) Internationale Anmeldenummer: PCT/EP2011/054905
(87) Internationale Veröffentlichungsnummer: WO 2011/121019

(56) Entgegenhaltungen:
- EP-A1- 0 551 626
- EP-B1- 1 575 633
- WO-A1-99/15150
- WO-A1-2008/033497
- WO-A2-2004/112713
- US-A1- 2002 198 315
- US-A1- 2005 037 050
- LI J ET AL: "Formation of nano-hydroxyapatite crystal in situ in chitosan-pectin polyelectrolyte complex network", MATERIALS SCIENCE AND ENGINEERING C, ELSEVIER SCIENCE S.A, CH, Bd. 30, Nr. 6, 31. März 2010 (2010-03-31), Seiten 795-803, XP002674116, ISSN: 0928-4931, DOI: 10.1016/J.MSEC.2010.03.011 [gefunden am 2010-06-03]
- TIYABOONCHAI ET AL: "Formulation and characterization of amphotericin B-chitosan-dextran sulfate nanoparticles", INTERNATIONAL JOURNAL OF PHARMACEUTICS, ELSEVIER BV, NL, Bd. 329, Nr. 1-2, 20. Dezember 2006 (2006-12-20), Seiten 142-149, XP005809185, ISSN: 0378-5173, DOI: 10.1016/J.IJPHARM.2006.08.013

## Beschreibung

Die Erfindung bezieht sich auf die Gebiete der Polymerchemie, der Pharmazie und der Medizin und betrifft ein Verfahren zur Herstellung eines Drug-Delivery-Systems auf der Basis von Polyelektrolytkomplexen, wie in den Ansprüchen definiert, welches beispielsweise als Bestandteil von Implantaten Arzneistoffe in der Umgebung des Implantates freisetzt.

Für die Bereitstellung von Freisetzungs- oder sogenannten Drug-Delivery-Systemen (DDS) mit Freisetzung von Arzneistoffen (AS) an biomedizinisch relevanten Positionen besteht einerseits großer Bedarf aber auch großer Handlungsbedarf. DDS wurden von den Anfängen um 1940 und werden bis heute kontinuierlich erforscht. Sie sind ein wichtiger Bestandteil bei der Überführung neuer AS in die klinische Praxis [.B.Malafaya, u.a. (2002): Curr. Opinion Solid State Mater. Sci. 6, 283-312 (Part I),. 297-312. (Part II)]

Neuartige wirkstoffbeladene Polymersysteme [H.Tamba, u.a. (2005). Adv. Drug Deliv. Rev. 57, 357-376.; G.B.Sukohorukov, u.a. (2005). Small 182, 194-200.] erscheinen dafür besonders aussichtsreich. In diesem Zusammenhang können zum einen klassische Systeme aus tensidischen oder (Co)polymer-Liposomen [H.Ringsdorf (1975). Polym. Sci. Polymer Symp. 51, 135-153. ;D.D. Lasic (1998). Medical applications of Liposomes, Papahadjopoulos D., Ed.; Elsevier.;M.Antonietti, S. Förster (2003). Advanced Materials 15, 1323-1333.] verwendet werden. Zum anderen ermöglichen Polyelektrolythohlkapseln [A.I.Petrov, u.a. (2005). Biotechnol. Prog. 21, 918-925; K.Köhler, u.a. (2004) Macromolecules 37, 9546-9550] den physikalischen Einschluss von Wirkstoffen.

Allgemein bekannt sind Polyelektrolyt-Komplexe (PEK). Diese werden durch Komplexierung von gegensinnig geladenen Polyelektrolyten (PEL) hergestellt. Bei dieser Komplexierung können die PEK in Form von dispergierten kugelförmigähnlichen PEK-Nanopartikeln gebildet werden. Diese PEK-Nanopartikel werden durch kontrolliertes Mischen von Polykation- und Polyanionlösungen in nichtstöchiometrischen molaren Verhältnissen erhalten, [V.A.Kabanov, u.a. (1984). Pure Appl. Chem. 56, 343-354; B.Philipp, u.a. (1989). Prog. Polym. Sci. 14, 91-172]. PEK-Nanopartikel bestehen aus einem eher hydrophoben ladungskompensierten Kern und einer hydrophilen Schale, die durch den jeweiligen Überschuss-PEL (Polykation oder Polyanion) gebildet wird. PEK-Nanopartikel sind unter anderem für die Oberflächenmodifizierung von technischen Substraten, [X.Feng, u.a. (2007) Biomacromolecules 8, 2161-2166] interessant. Dabei können preferentiell bereits vorgebildete Nanopartikel an das entsprechende Substrat gebunden werden oder der Komplexierungsschritt erfolgt im Beisein des Substrats.

Großes Potential der PEC-Partikel wird für Nanoträgersysteme in der Volumenphase im Bereich der Biomedizin und Pharmazie gesehen [P.Dubin, u.a. (eds.) (1994). Macromolecular Complexes in Chemistry and Biology, Springer-Verlag] In diesem Zusammenhang sind Arbeiten von Tiyaboonchai [W.Tiyaboonchai, u.a. (2001) J. Pharm. Sci. 90, 902-914] bekannt, in welchen die Formulierung und Charakterisierung von Nanopartikeln aus Poly(ethylenimin) (PEI), Dextransulfat (DS) und Amphotericin B (AmB) beschrieben ist. AmB ist ein antifungaler AS bei systemischen Pilzinfektionen, wird aber aufgrund der geringen Wasserlöslichkeit nicht vom Gastrointestinaltrakt aufgenommen. Ebenfalls ist die Entwicklung eines Brustkrebstherapeutikums auf der Basis von Mikropartikeln aus Chitosan/Alginat untersucht worden [G.Coppi, u.a. (2009). Int. J. Pharmaceut. 367, 127-132], an welchen die Aufnahme und die zeitabhängige Abgabe von Tamoxifen geprüft wurde. Weiterhin wurden die Aufnahme von Salbutamol, einem Asthmatherapeutikum, an Komplexpartikeln aus den zwei entgegengesetzt geladenen Proteinen Gelatine A und Gelatine B im Submikrometerbereich und dessen verzögerte Abgabe in gastrosimulierende Flüssigkeiten beschrieben [A.Tiwari, u.a. (2009). Biomacromolecules 10, 184-189]. Salbutamol wurde während und nach der Komplexierung zugegeben. Als eine wichtige Triebkraft der Freigabe wurde die osmotische Druckdifferenz zwischen Partikelinnerem und Umgebung genannt. Auch interessant in Bezug auf die Erfindung sind Arbeiten zur Nutzung von PEK-Partikeln als Trägersysteme generell für Proteine [W.Ouyang, u.a. (2006). Macromol. Biosci. 6, 929-941], speziell für Wachstumsfaktoren (VEGF) (Chitosan/Dextransulfat) [M.Huang, u.a. (2007) Biomacromolecules 8, 1607-1614] aber auch für Plasmid-DNA [W.Tiyaboonchai, u.a. (2003) Eur. J. Pharm. Sei. 19, 191-202].

Nach der EP 0 551 626 A1 wird ein Polymer enthaltendes thermoreversibles Gel als Träger für flüssige Pharmazeutika eingesetzt.

Bei Li wird kristallines Nano-Hydroxyapatit in ein Chitosan-Pektin Polyelektrolyt-Komplex-Netzwerk eingebaut [Li J u.a. (2010) Materials Science and Engineering C, Elsevier Science S.A., CH, Bd. 30, Nr. 6, S. 795-803 "Formation of nanohydroxapatite crystal in situ in chitosan-pectin polyelectrolyte complex network"].

Tiyaboonchai et al (2006) International Journal of Pharmaceutics, Bd 329, Nr. 1-2, 142-149, beschreibt einen Prozess zur Herstellung von Amphotericin B-Chitosan-Dextransulfat-Nanopartikeln durch Polyelektrolyt Komplexierung für die parenterale Anwendung. Nachteile bei den bekannten technischen Lösungen für Drug-Delivery-Systeme bestehen vor allem in der noch nicht ausreichenden Zielgenauigkeit bei lokal gesteuerten Freisetzungen von AS an den gewünschten Stellen sowie in der Geschwindigkeit und Menge der Abgabe der AS.

Die Aufgabe der vorliegenden Erfindung besteht in der Angabe eines Verfahrens zur Herstellung eines Drug-Delivery-Systems auf der Basis von Polyelektrolytkomplexen, welches auf einfache und gut reproduzierbare Art und Weise ein Drug-Delivery-System erzeugt, welches lokal gezielt und steuerbar verzögert Arzneistoffe frei gibt. Die Aufgabe wird durch die in den Ansprüchen angegebene Erfindung gelöst. Vorteilhafte Ausgestaltungen sind Gegenstand der Unteransprüche. Offenbart sind Verfahren zur Herstellung eines Drug-Delivery-Systems auf der Basis von Polyelektrolytkomplexen werden Polyanionen und Polykationen in einem nichtstöchiometrischen Verhältnis, bezogen auf die geladenen Monomereinheiten in einer Flüssigkeit gemischt, wobei ein, zwei oder mehrere Arzneistoffe entweder vor, während oder nach der Mischung der Polyelektrolyte zugegeben werden, oder ein, zwei oder mehrere ladungstragende Arzneistoffe und ein entgegengesetzt geladener Polyelektrolyt gemischt werden, und nach der Mischung der entstandene Polyelektrolyt-Komplex auf die Oberfläche oder den Oberflächenbereich eines medizinischen Mittels aufgebracht wird oder auf die Oberfläche oder den Oberflächenbereich direkt an dem Ort positioniert wird, wo der Arzneistoff freigesetzt werden soll.

Vorteilhafterweise werden als Polyanionen anionische Polypeptide, Poly(L-Glutaminsäure), Poly(D-Glutaminsäure), anionische Polysaccharide, Dextransulfat, Heparin, Cellulosesulfat, Carboxymethylcellulose, Carboxymethylstärke, Alginat, Carrageenan, Xanthan, Hyaluronsäure, Poly(acrylsäure) eingesetzt.

Ebenfalls vorteilhafterweise werden als Polykationen kationische Polypeptide, (Poly(L-Lysin), Poly(D-Lysin), kationische Polysaccharide, Dietyhlaminoethyl(DEAE)dextran, Chitosan, kationische Stärke, Poly(methylen-co-guanidin), Poly(ethylenimin) eingesetzt.

Weiterhin vorteilhafterweise wird als Flüssigkeit Wasser mit eingestelltem pH-Wert, lonenstärke und Temperatur, eingesetzt.

Und auch vorteilhafterweise werden Polyanionen und Polykationen in einem dem stöchiometrischen Verhältnis von 1, bezogen auf die ladungstragenden Monomereinheiten, möglichst nahe kommenden Wert gemischt.

Ebenfalls vorteilhaft ist es, wenn Polyanionen und Polykationen in einem nichtstöchiometrischen Verhältnis von (0,5 bis < 1) bis (>1 bis 2), vorteilhafterweise (0,9 bis < 1) bis (>1 bis 1,1), gemischt werden.

Und auch vorteilhaft ist es, wenn das nichtstöchiometrische Verhältnis der Polykationen und Polyanionen über unterschiedliche Volumina bei gleicher Konzentration der Polykationen und Polyanionen bezogen auf die ladungstragenden Monomereinheiten realisiert wird.

Vorteilhaft ist es weiterhin, wenn ein Arzneistoff vor der Mischung einer Polyelektrolytlösung, während der Mischung oder nach der Mischung der Polyelektrolytlösungen zugegeben wird.

Ebenfalls vorteilhaft ist es, wenn für die Herstellung einer AS-PEK-Dispersion ein ladungstragender Arzneistoff einer der Polyelektrolytlösungen zugegeben wird.

Weiterhin vorteilhaft ist es, wenn der Arzneistoff in einer Menge zugegeben wird, die einem stöchiometrischen Verhältnis zu den geladenen Monomereinheiten eines entgegengesetzt geladenen Polyelektrolyten von kleiner als 1 entspricht.

Und auch vorteilhaft ist es, wenn einfache oder mehrfache anionische und/oder kationische Ladungen tragende Arzneistoffe, vorteilhafterweise Bisphosphonate (BP), oder ladungstragende Antibiotika, vorteilhafterweise Streptomycin, Gentamycin, Penicillin und/oder Nystatin eingesetzt werden.

Von Vorteil ist es auch, wenn ungeladene Arzneistoffe eingesetzt werden.

Weiterhin von Vorteil ist es, wenn als Arzneistoffe Protonenpumpeninhibitoren (PPI), wie beispielsweise Pantoprazol und/oder Statine (STA), wie beispielsweise Pravastatin, und/oder Proteasominhibitoren (PSI), wie beispielsweise Bortezomib, eingesetzt werden.

Ebenso ist es von Vorteil, wenn zwei und mehrere Arzneistoffe eingesetzt werden und diese gleich oder unterschiedlich verzögert freigesetzt werden.

Und auch von Vorteil ist es, wenn die Mischung der Polyelektrolyte durch präparative Prozessparameter, wie Zugabereihenfolge, Rührgeschwindigkeit, konsekutive Schritte aus Zentrifugieren-Dekantieren-Redispergieren realisiert wird.

Vorteilhaft ist es auch, wenn bei der Herstellung der nichtstöchiometrischen Mischungen aus Polyanion/Polykation und aus Polyelektrolyt/Arzneistoff, bezogen auf die geladenen Monomereinheiten der Polyelektrolyte und die geladenen Gruppen des AS, die Überschusskomponente vorgelegt und die Unterschusskomponente zudosiert wird.

Und auch vorteilhaft ist es, wenn eine PEK-Dispersion hergestellt wird, die überwiegend aus monomodal verteilten nanoskaligen Partikeln (Polyelektrolyt-Komplex-Partikeln - PEK-Partikel) mit definierter Partikelgröße und definiertem Ladungsvorzeichen besteht.

Weiterhin von Vorteil ist es, wenn PEK-Dispersionen aus polymodal verteilten nanoskaligen Partikeln mit Partikeldurchmessern im Bereich von 10 bis 1000 nm eingesetzt werden.

Und ebenfalls vorteilhaft ist es, wenn PEK-Dispersionen hergestellt werden, deren PEK-Partikel latexähnliche Eigenschaften aufweisen.
Vorteilhafterweise wird der Polyelektrolyt-Komplex mittels Adsorption, Tauchen, Sprühen, Streichen, Überfließen/Überströmen, auf die Oberfläche von medizinischen Mitteln aufgebracht und die aufgebrachte Schicht durch Temperaturerhöhung getrocknet und das Lösungsmittel entfernt, wodurch eine stabile oder irreversibel oberflächengebundene Schicht hergestellt wird.

Weiterhin vorteilhafterweise wird nach dem Aufbringen des Polyelektrolyt-Komplexes die aufgebrachte Schicht mit Wasser gespült.

Und auch von Vorteil ist es, wenn die Flüssigkeitskomponente der aufgebrachten Schicht durch Erniedrigung des Drucks (Vakuum) entfernt wird.

Von Vorteil ist es auch, wenn als medizinische Mittel Implantate, Knochenersatzmaterialien, Wundverschlüsse, Nahtmaterialien eingesetzt werden.

Und auch vorteilhafterweise wird die direkte Positionierung des Polyelektrolyt-Komplexes durch lokales Einspritzen realisiert.

Ebenfalls vorteilhaft ist es, wenn einer der beiden oder beiden Polyelektrolytlösungen oder der Arzneistofflösung oder der Polyelektrolyt/Arzneistoff-Mischung oder der durch Mischung erhaltenen PEK-Dispersion anorganische Salze, wie beispielsweise Calziumdichlorid, und/oder Puffersubstanzen, wie beispielsweise Citratpuffer, zugegeben werden.

Mit der erfindungsgemäßen Lösung wird es erstmals möglich, durch den Einsatz von Polyelektrolytkomplexen Arzneistoffe lokal gezielt und steuerbar verzögert freizusetzen. Offenbart ist ebenfalls eine Option, bei der Polykationen und Polyanionen in einem nichtstöchiometrischen Verhältnis gemischt und der Arzneistoff (AS) vorteilhafterweise während der Mischung zugegeben. Dabei müssen die Konzentration, der pH-Wert, die Ionenstärke und das molare Verhältnis der Polyelektrolyte und des AS aufeinander abgestimmt sein, sodass ein definiertes stöchiometrisches Verhältnis bezogen auf die Monomereinheiten der beiden Polyelektrolyte eingestellt wird. Bei dem Mischen der Polykationen und -anionen in einer Flüssigkeit in einem, vorteilhafterweise leicht, nichtstöchiometrischen Verhältnis entsteht ein mindestens schwach geladener Polyelektrolyt-Komplex (PEK). Die Nettoladung des Polyelektrolytkomplexes wird durch die Überschusskomponente bestimmt, so dass das Polyanion oder anionische Monomereinheiten im Überschuss zu negativ geladenen PEK-Partikeln und das Polykation oder kationische Momomereinheiten im Überschuss zu positiv geladenen PEK-Partikeln führt. Vorteilhafterweise wird die Polyelektrolyt-Lösung mit den überschüssigen geladenen Monomereinheiten (Überschuss-Polyelektrolyt) vorgelegt und die Polyelektrolyt-Lösung mit den unterschüssigen entgegengesetzt geladenen Monomereinheiten (Unterschuss-Polyelektrolyt) zudosiert.

Je nachdem, welche Ladung die eingesetzten AS aufweisen, ist der entstehende Polyelektrolyt-Komplex vorteilhaft so aufgebaut, dass er mindestens schwach gegensätzlich zu den AS geladen ist. Im Falle, dass der AS kationische oder anionische Ladungen trägt, ist der AS dann bereits Bestandteil des Komplexes. Vorteilhafterweise sollte der entstehende Polyelektrolytkomplex mindestens eine schwache gegensätzliche Ladung zum AS aufweisen, damit der AS langzeitiger im Komplex festgehalten werden kann.

Polyelektrolyt-Komplexe liegen in der Regel als wässrige Dispersionen vor, welche im Wesentlichen aus nanoskaligen Partikeln (Polyelektrolyt-Komplex-Partikeln - PEK-Partikel) bestehen, deren wahlweise positive oder negative Nettoladung durch das Mischungsverhältnis eingestellt werden kann. PEK-Partikel haben eine weiche, latexähnliche Struktur mit einem ladungskompensierten eher hydrophoben Kern und einer geladenen hydrophilen Schale (Kern/Schale-Partikel) und sind über Methoden, wie der dynamischen Lichtstreuung oder der in-situ-ATR-FTIR-Spektroskopie in einer speziellen Durchflusszelle, nachweisbar (M. Müller, u.a. Langmuir 2005, 21, 7044-7051). Deren Partikeldurchmesser kann durch Struktur und Medienparameter im Bereich von 10 - 1000 nm eingestellt werden und beträgt unter Standardbedingungen in der Regel 150-300 nm. Frisch hergestellte PEK-PartikelDispersion weisen in der Regel verschieden breite Größenverteilungen auf, welche optional durch konsekutive Schritte aus Zentrifugation, Separation und Redispergierung erheblich verschmälert werden können.

Bei der Herstellung von AS-beladenen PEK-Partikeln wird der AS sowohl in den Partikelkern eingebaut als auch in die Partikelschale angelagert.
Erfindungsgemäß werden die AS-beladenen PEK-Partikel auf biomedzinisch relevante Oberflächen des medizinischen Mittels aufgebracht. Die Abscheidung kann durch Eintauchen und Adsorption, Überströmen, Ausstreichen oder Aufsprühen jeweils gefolgt von einem Trocknungsschritt erfolgen.
Dabei bilden die PEK-Partikel nach dem Aufbringen auf die Oberfläche des medizinischen Mittels und Entfernung der Flüssigkeit eine latex-ähnliche Schicht, die aus den immer noch relativ weichen oder teilweise fusionierten PEK-Partikeln mit den AS besteht. Falls das medizinische Mittel geladene funktionelle Gruppen an der Oberfläche besitzt, werden vorteilhafterweise die dazu entgegengesetzt geladenen PEK-Partikel aufgebracht.
Nach Einbringung eines so modifizierten medizinischen Mittels an die gewünschte Stelle und Umgebung, beispielsweise eines Implantates in ein Gewebe, wird der AS freigesetzt. Dabei wird der an der Schale der PEK-Partikel befindliche AS im Milieu der Umgebung (u.a. extrazelluläre Gewebeflüssigkeit, Blut) des medizinischen Mittels zuerst freigesetzt. Danach werden die sich weiter im Inneren der PEK-Partikel befindlichen AS verzögert freigesetzt. Bei der Freisetzung spielen diffussive und osmotische Prozesse eine Rolle.

Die steuerbar verzögerte Freisetzung des AS aus dem PEK wird erfindungsgemäß dadurch erreicht, dass die AS sich teilweise im PEK-Partikelkern und teilweise an der Partikelschale befinden.

Als Freisetzungsmedien werden vorteilhafterweise wässrige Lösungen, nach sinnvoller Einstellung und Wahl von Salzkonzentration, Puffersubstanztyp und pH-Wert eingesetzt.

Die nach der Aufbringung auf die Oberfläche des medizinischen Mittels entstehenden PEK-Partikel/AS-Schichten sind stabil gegen Ablösung in Wasser und verschiedenen Pufferlösungen. Dadurch stellen sie keine negative Beeinflussung für das umgebende Gewebe dar und können beispielsweise auch mit dem medizinischen Mittel später wieder entfernt werden.

Die Wirkungsweise einer solchen abgeschiedenen AS-beladenen PEK-Dispersion ergibt sich aus dem Vergleich zum abgeschiedenen reinen Wirkstoff: Für den im und am PEK-Partikel gebundenen AS ergibt sich eine im Vergleich zur reinen AS-Schicht verzögerte Freisetzungskinetik in Kontakt zum wässrigen Freisetzungsmedium. Besonders vorteilhaft ist bei der Abscheidung auf medizinischen Mitteln, dass die abgeschiedenen Schichten aus PEK-Partikel/AS im Wesentlichen fest auf der Oberfläche haften und irreversibel abgeschieden sind.
Bei medizinischen implantatgestützten regenerativen Operationen werden die PEK-Partikel/AS-Dispersionen direkt durch Eintauchen, Sprühen oder Ausstreichen mit nachfolgender Trocknung auf das Implantat aufgebracht. Dabei kann einstellbar bereits initial ein Anteil des AS und der Rest postoperativ verzögert freigesetzt werden.
Die Freisetzung des AS kann durch Variation der kationischen und anionischen Polymersysteme (u.a. Struktur, Ladungsdichte, Molekulargewicht), das stöchiometrische Mischungsverhältnis, das Verhältnis AS/Polyelektrolyt, die Verwendung von Hilfsstoffen wie niedermolekulare Salzionen und durch weitere Änderungen im Präparationsprotokoll individuell an die biomedizinische Anwendung angepasst werden.

Die Vorteile des erfindungsgemäß hergestellten Drug-Delivery-Systems gegenüber den bekannten Systemen, wie Liposomen oder Hohlkapseln, sind folgende:
- Größenskalierbarkeit der PEK-Partikel von 10 - 1000 nm über Polyelektrolyt- und Partikel-Dimension und Medienparameter (Konzentration, pH, Ionenstärke);
- Adhärierbarkeit der PEK/AS-Schichten durch Adsorption (nass) oder Verfilmung (trocken) unter Erhalt von Form und Funktion am medizinischen Mittel (Therapie) oder auch an Modellsubstraten (Screening, Kombinatorik);
- Einstellbares Bindungs- oder Freisetzungspotential des AS durch Art der Wechselwirkung (Elektrostatik oder physikalischer Einschluss), Polymer- oder Partikelstruktur und -größe;
- Variabilität hinsichtlich des AS.

Nachfolgend wird die Erfindung an einem Ausführungsbeispiel näher erläutert.

### Beispiel 1

Als AS wurde ein Bisphosphonat (Pamidronat), ein zugelassenes Mittel zur Osteoporostherapie, eingesetzt. Die AS beladene PEK-Dispersion wurde durch Mischen einer 0.001 M Dextransulfatlösung (Substitutionsgrad DS ≈ 3) mit einer 0.001 M Poly(L-lysin)-(PLL)-Lösung, welche vorher mit einer 0.01 M Bisphosphonat-(BP)-Lösung und einer 0.01 m Calziumdichlorid-(CaCl₂)-Lösung, beide in molaren Verhältnissen 4:1 (PLL/BP und PLL/CaCl₂), gemischt wurde, im Volumenverhältnis 3:10 hergestellt. Bei der Herstellung der nichtstöchiometrischen Mischungen aus der Dextransulfatlösung und der Poly(L-lysin)-Lösung (Polyanion und Polykation) und aus Polyelektrolyt/AS wurde jeweils die Überschusskomponente (Poly(L-lysin)) vorgelegt und die Unterschusskomponente (Dextransulfat) zudosiert. 50 Mikroliter dieser AS-beladenen PEK-Dispersion wurden auf ein anorganisches planares Modellsubstrat aus Germanium ausgestrichen und im Trockenschrank bei 50°C eingetrocknet. Die Messung der Freisetzung des Bisphosphonats und der Stabilität der PEK-Trägerschicht erfolgte über spektroskopische Verfahren. Nach 24 Stunden ergab sich eine relative Abnahme des Bisphosphonatgehalts der PEK/AS-Schicht von ca. 50%. Die PEK-Trägerschicht blieb nach Freisetzung des Bisphosphonats stabil auf dem Substrat gebunden. Für eine wie im Fall von PEK/AS auf demselben Modellsubstrat ausgestrichene und eingetrocknete Schicht des reinen Bisphosphonats ergab sich nach 1 Minute eine sofortige Ablösung und damit eine relative Abnahme des Bisphosphonatgehalts von ca. 100%.

## Patentansprüche

1. Verfahren zur Herstellung eines Drug-Delivery-Systems auf der Basis von Polyelektrolytkomplexen, bei dem Polyanionen und Polykationen in einem nichtstöchiometrischen Verhältnis, bezogen auf die geladenen Monomereinheiten, in einer Flüssigkeit gemischt werden, wobei ein Arzneistoff vor der Mischung der Polyelektrolyte zugegeben wird, wobei als Arzneistoff Protonenpumpeninhibitoren (PPI) oder Statine (STA) oder Proteasominhibitoren (PSI) oder Bisphosphonate (BP) oder ladungstragende Antibiotika eingesetzt werden, und nach der Mischung der entstandene Polyelektrolyt-Komplex auf die Oberfläche oder den Oberflächenbereich eines medizinischen Mittels aufgebracht wird.

2. Verfahren nach Anspruch 1, bei dem als Polyanionen anionische Polypeptide, Poly(L-Glutaminsäure), Poly(D-Glutaminsäure), anionische Polysaccharide, Dextransulfat, Heparin, Cellulosesulfat, Carboxymethylcellulose, Carboxymethylstärke, Alginat, Carrageenan, Xanthan, Hyaluronsäure, Poly(acrylsäure) eingesetzt werden.

3. Verfahren nach Anspruch 1, bei dem als Polykationen kationische Polypeptide, (Poly(L-Lysin), Poly(D-Lysin), kationische Polysaccharide, Dietyhlaminoethyl(DEAE)dextran, Chitosan, kationische Stärke, Poly(methylen-co-guanidin), Poly(ethylenimin) eingesetzt werden.

4. Verfahren nach Anspruch 1, bei dem als Flüssigkeit Wasser mit eingestelltem pH-Wert, Ionenstärke und Temperatur, eingesetzt wird.

5. Verfahren nach Anspruch 1, bei dem Polyanionen und Polykationen in einem dem stöchiometrischen Verhältnis von 1, bezogen auf die ladungstragenden Monomereinheiten, möglichst nahe kommenden Wert gemischt werden, wobei vorteilhafterweise Polyanionen und Polykationen in einem nichtstöchiometrischen Verhältnis von (0,5 bis < 1) bis (>1 bis 2), noch vorteilhafterweise (0,9 bis < 1) bis (>1 bis 1,1) gemischt werden.

6. Verfahren nach Anspruch 1, bei dem das nichtstöchiometrische Verhältnis der Polykationen und Polyanionen über unterschiedliche Volumina bei gleicher Konzentration der Polykationen und Polyanionen bezogen auf die ladungstragenden Monomereinheiten realisiert wird.

7. Verfahren nach Anspruch 1, bei dem der Arzneistoff in einer Menge zugegeben wird, die einem stöchiometrischen Verhältnis zu den geladenen Monomereinheiten eines entgegengesetzt geladenen Polyelektrolyten von kleiner als 1 entspricht.

8. Verfahren nach Anspruch 1, bei dem ungeladene Arzneistoffe eingesetzt werden.

9. Verfahren nach Anspruch 1, bei dem als Arzneistoffe Pantoprozol (PPI) oder Pravastatin (STA) oder Bortezomib (PSI) eingesetzt werden.

10. Verfahren nach Anspruch 1, bei dem Arzneistoffe eingesetzt werden und diese gleich oder unterschiedlich verzögert freigesetzt werden.

11. Verfahren nach Anspruch 1, bei dem die Mischung der Polyelektrolyte durch präparative Prozessparameter, wie Zugabereihenfolge, Rührgeschwindigkeit, und/oder konsekutive Schritte aus Zentrifugieren-Dekantieren-Redispergieren realisiert wird.

12. Verfahren nach Anspruch 1, bei dem bei der Herstellung der nichtstöchiometrischen Mischungen aus Polyanion/Polykation und aus Polyelektrolyt/Arzneistoff, bezogen auf die geladenen Monomereinheiten der Polyelektrolyte und die geladenen Gruppen des AS, die Überschusskomponente vorgelegt und die Unterschusskomponente zudosiert wird.

13. Verfahren nach Anspruch 1, bei dem eine PEK-Dispersion hergestellt wird, die überwiegend aus monomodal verteilten nanoskaligen Partikeln (Polyelektrolyt-Komplex-Partikeln - PEK-Partikel) besteht.

14. Verfahren nach Anspruch 1, bei dem PEK-Dispersionen aus polymodal verteilten nanoskaligen Partikeln mit Partikeldurchmessern im Bereich von 10 bis 1000 nm eingesetzt werden.

15. Verfahren nach Anspruch 1, bei dem PEK-Dispersionen hergestellt werden, deren PEK-Partikel latexähnliche Eigenschaften aufweisen.

16. Verfahren nach Anspruch 1, bei dem der Polyelektrolyt-Komplex mittels Adsorption, Tauchen, Sprühen, Streichen, Überfließen/Überströmen, auf die Oberfläche von medizinischen Mitteln aufgebracht wird und die aufgebrachte Schicht durch Temperaturerhöhung getrocknet und das Lösungsmittel entfernt wird, wodurch eine stabile oder irreversibel oberflächengebundene Schicht hergestellt wird.

17. Verfahren nach Anspruch 1, bei dem nach dem Aufbringen des Polyelektrolyt-Komplexes die aufgebrachte Schicht mit Wasser gespült wird.

18. Verfahren nach Anspruch 1, bei dem die Flüssigkeitskomponente der aufgebrachten Schicht durch Erniedrigung des Drucks entfernt wird.

19. Verfahren nach Anspruch 1, bei dem als medizinische Mittel Implantate, Knochenersatzmaterialien, Wundverschlüsse, Nahtmaterialien eingesetzt werden.

20. Verfahren nach Anspruch 1, bei dem die direkte Positionierung des Polyelektrolyt-Komplexes durch lokales Einspritzen auf die Oberfläche oder den Oberflächenbereich eines medizinischen Mittels realisiert wird.

21. Verfahren nach Anspruch 1, bei dem einer der beiden oder beiden Polyelektrolytlösungen oder der Arzneistofflösung oder der Polyelektrolyt/Arzneistoff-Mischung oder der durch Mischung erhaltenen PEK-Dispersion anorganische Salze, wie beispielsweise Calziumdichlorid, und/oder Puffersubstanzen, wie beispielsweise Citratpuffer, zugegeben werden.

## Claims

1. Method for producing a drug delivery system on the basis of polyelectrolyte complexes, in which polyanions and polycations are mixed in a liquid in a non-stoichiometric ratio relative to the charged monomer units, wherein a drug substance is added prior to the mixing of the polyelectrolytes, wherein proton pump inhibitors (PPI) or statins (STA) or proteasome inhibitors (PSI) or bisphosphonates (BP) or charge-bearing antibiotics are used as drug substance, and after the mixing the resulting polyelectrolyte complex is applied to the surface or to the surface region of a medical means.

2. Method according to Claim 1, in which anionic polypeptides, poly(L-glutamic acid), poly(D-glutamic acid), anionic polysaccharides, dextran sulphate, heparin, cellulose sulphate, carboxymethyl cellulose, carboxymethyl starch alginate, carrageenan, xanthan, hyaluronic acid, poly(acrylic acid) are used as polyanions.

3. Method according to Claim 1, in which cationic polypeptides, poly(L-lysine), poly(D-lysine), cationic polysaccharides, diethylaminoethyl (DEAE)-dextran, chitosan, cationic starch, poly (methylene-co-guanidine), poly(ethylenimine) are used as polycations.

4. Method according to Claim 1, in which water with adjusted pH, ionic strength and temperature is used as a liquid.

5. Method according to Claim 1, in which polyanions and polycations are mixed in a value coming as close as possible to the stoichiometric ratio of 1 relative to the charge-bearing monomer units, wherein polyanions and polycations are mixed in a non-stoichiometric ratio of advantageously (0.5 to < 1) to (>1 to 2), more advantageously (0.9 to < 1) to (>1 to 1.1).

6. Method according to Claim 1, in which the non-stoichiometric ratio of polycations and polyanions is achieved via different volumes with the same concentration of polycations and polyanions relative to the charge-bearing monomer units.

7. Method according to Claim 1, in which the drug substance is added in an amount that corresponds to a stoichiometric ratio to the charged monomer units of an oppositely charged polyelectrolyte of less than 1.

8. Method according to Claim 1, in which uncharged drug substances are used.

9. Method according to Claim 1, in which pantoprazole (PPI) or pravastatin (STA) or bortezomib (PSI) are used as drug substances.

10. Method according to Claim 1, in which drug substances are used and the latter are released in an identically or differently delayed manner.

11. Method according to Claim 1, in which the mixing of the polyelectrolytes is achieved by preparative process parameters such as order of addition, stirring speed, and/or consecutive steps of centrifuging-decanting-redispersing.

12. Method according to Claim 1, in which during the production of the non-stoichiometric mixtures of polyanions/polycations and of polyelectrolytes/drug substances relative to the charged monomer units of the polyelectrolytes and the charged groups of the drug substance, the excess component is initially introduced and the deficient component is added.

13. Method according to Claim 1, in which a PEC dispersion is produced, which is composed chiefly of monomodally distributed nanoscale particles (polyelectrolyte complex particles - PEC particles).

14. Method according to Claim 1, in which PEC dispersions of polymodally distributed nanoscale particles with particle diameters ranging from 10 to 1000 nm are used.

15. Method according to Claim 1, in which PEC dispersions are produced, the PEC particles of which have latex-like properties.

16. Method according to Claim 1, in which the polyelectrolyte complex is applied to the surface of medical means by means of adsorption, immersion, spraying, brushing, flowing over/streaming over, and the applied layer is dried by raising the temperature and the solvent is removed, and a stable or irreversibly surface-bonded layer is thus produced.

17. Method according to Claim 1, in which after the application of the polyelectrolyte complex the applied layer is rinsed with water.

18. Method according to Claim 1, in which the liquid component of the applied layer is removed by lowering the pressure.

19. Method according to Claim 1, in which implants, bone replacement materials, wound closures, suture materials are used as medical means.

20. Method according to Claim 1, in which the polyelectrolyte complex is directly positioned on the surface or surface region of the medical means by means of local injection.

21. Method according to Claim 1, in which inorganic salts, such as calcium chloride, and/or buffer substances, such as citrate buffer, can be added to one of the two or to both polyelectrolyte solutions or to the drug substance solution or to the polyelectrolyte-drug substance mixture or to the PEC dispersion obtained by mixing.

## Revendications

1. Procédé pour la fabrication d'un système de libération de médicament à base de complexes polyélectrolytiques, dans lequel des polyanions et des polycations sont mélangés dans un liquide dans un rapport non stoechiométrique, par rapport aux unités monomères chargées, un médicament étant ajouté avant le mélange des polyélectrolytes, des inhibiteurs de pompe à protons (PPI) ou des statines (STA) ou des inhibiteurs de protéasome (PSI) ou des bisphosphonates (BP) ou des antibiotiques portant des charges étant utilisés comme médicament et, après le mélange, le complexe polyélectrolytique formé étant appliqué sur la surface ou la zone surfacique d'un agent médical.

2. Procédé selon la revendication 1, dans lequel on utilise, comme polyanions, des polypeptides anioniques, le poly(acide L-glutamique), le poly(acide D-glutamique), des polysaccharides anioniques, le sulfate de dextran, l'héparine, le sulfate de cellulose, la carboxyméthylcellulose, le carboxyméthylamidon, l'alginate, le carraghénane, le xanthane, l'acide hyaluronique, le poly(acide acrylique).

3. Procédé selon la revendication 1, dans lequel on utilise, comme polycations, des polypeptides cationiques, la poly(L-lysine), la poly(D-lysine), les polysaccharides cationiques, le diéthylaminoéthyl(DEAE)dextran, le chitosane, l'amidon cationique, le poly(méthylène-co-guanidine), le poly(éthylène-imine).

4. Procédé selon la revendication 1, dans lequel on utilise, comme liquide, de l'eau présentant une valeur de pH, une force ionique et une température réglées.

5. Procédé selon la revendication 1, dans lequel des polyanions et des polycations sont mélangés à une valeur la plus proche possible du rapport stoechiométrique de 1, par rapport aux unités monomères portant une charge, les polyanions et les polycations étant avantageusement mélangés dans un rapport non stoechiométrique de (0,5 à < 1) à (> 1 à 2), encore plus avantageusement de (0,9 à < 1) à (> 1 à 1, 1).

6. Procédé selon la revendication 1, dans lequel le rapport non stoechiométrique des polycations et des polyanions est réalisé via des volumes différents à une concentration identique en polycations et en polyanions par rapport aux unités monomères portant une charge.

7. Procédé selon la revendication 1, dans lequel le médicament est ajouté en une quantité qui correspond à un rapport stoechiométrique inférieur à 1, par rapport aux unités monomères chargées d'un polyélectrolytique chargé de manière opposée.

8. Procédé selon la revendication 1, dans lequel des médicaments non chargés sont utilisés.

9. Procédé selon la revendication 1, dans lequel on utilise, comme médicaments, du pantoprazol (PPI) ou de la pravastatine (STA) ou du bortézomib (PSI).

10. Procédé selon la revendication 1, dans lequel des médicaments sont utilisés et ceux-ci sont libérés en même temps ou de manière retardée, différente.

11. Procédé selon la revendication 1, dans lequel le mélange des polyélectrolytes est réalisé par des paramètres de procédé préparatif, tels que l'ordre d'addition, la vitesse d'agitation et/ou des étapes consécutives de centrifugation - décantation - redispersion.

12. Procédé selon la revendication 1, dans lequel, lors de la préparation des mélanges non stoechiométriques de polyanion/polycation et de polyélectrolyte/médicament, par rapport aux unités monomères chargées des polyélectrolytes et aux groupes chargés du médicament, le composant en excès est disposé au préalable et le composant inférieur à la quantité stoechiométrique est ajouté en dosant.

13. Procédé selon la revendication 1, dans lequel on prépare une dispersion de CPE qui est principalement constituée par des particules nanométriques réparties de manière monomodale (particules de complexe polyélectrolytique - particules CPE).

14. Procédé selon la revendication 1, dans lequel des dispersions de CPE constituées par des particules nanométriques réparties de manière polymodale présentant des diamètres de particule dans la plage de 10 à 1000 nm sont utilisées.

15. Procédé selon la revendication 1, dans lequel des dispersions de CPE sont préparées, dont les particules de CPE présentent des propriétés analogues à un latex.

16. Procédé selon la revendication 1, dans lequel le complexe polyélectrolytique est appliqué par adsorption, immersion, pulvérisation, enduction, recouvrement/submersion, à la surface d'agents médicaux et la couche appliquée est séchée par augmentation de température et le solvant est éliminé, suite à quoi une couche stable ou liée de manière irréversible à la surface est réalisée.

17. Procédé selon la revendication 1, dans lequel, après l'application du complexe polyélectrolytique, la couche appliquée est rincée à l'eau.

18. Procédé selon la revendication 1, dans lequel le composant liquide de la couche appliquée est éliminé par diminution de la pression.

19. Procédé selon la revendication 1, dans lequel on utilise, comme agent médical, des implants, des matériaux de remplacement osseux, des pansements de plaie, des matériaux de suture.

20. Procédé selon la revendication 1, dans lequel le positionnement direct du complexe polyélectrolytique est réalisé par injection locale à la surface ou la zone surfacique d'un agent médical.

21. Procédé selon la revendication 1, dans lequel une des deux solutions polyélectrolytiques ou les deux ou la solution de médicament ou le mélange polyélectrolyte/médicament ou la dispersion de CPE obtenue par le mélange sont additionnés de sels inorganiques, tels que par exemple le dichlorure de calcium et/ou de substances tampon, telles que par exemple un tampon citrate.
